# EUROPEAN PATENT APPLICATION

(11) **EP 3 730 622 A1**
(43) Date of publication of application: **28.10.2020**
(21) Application number: 18892535.8
(22) Date of filing: 10.12.2018
(51) Int. Cl.: C12P 7/10, C12P 19/02, C12N 9/24

(54) **METHOD FOR ENZYMATICALLY PRODUCING BIOETHANOL USING CELLULOSIC BIOMASS AS STARTING MATERIAL**

(30) Priority: 19.12.2017 JP 2017242955
(71) Applicant: Kawasaki Jukogyo Kabushiki Kaisha, Hyogo 650-8670 (JP)
(72) Inventor: NISHINO, Takashi, Hyogo 650-8670 (JP); IZUMI, Noriaki, Hyogo 650-8670 (JP); TAJIRI, Hironori, Hyogo 650-8670 (JP); TSUJITA, Shoji, Hyogo 650-8670 (JP); ODA, Asuka, Hyogo 650-8670 (JP); MASAMOTO, Manabu, Hyogo 650-8670 (JP); WARATANI, Yusuke, Hyogo 650-8670 (JP)
(74) Representative: Leinweber & Zimmermann
(86) International application number: PCT/JP2018/045299
(87) International publication number: WO 2019/124143

(57) **Abstract**

An object of the present invention is to provide a bioethanol production method using lignocellulosic biomass as a raw material, the method being adapted to increase the ethanol concentration of a fermentation liquid obtained in a fermentation step and reduce the distillation load without having to use specialized equipment in solubilizing the biomass by enzymatic hydrolysis of cellulose contained in the biomass. When a solid residue of the cellulosic biomass, from which hemicellulose has been removed, is mixed with an aqueous solution containing a cellulose-hydrolyzing enzyme in a reaction vessel, ethanol is added in an amount of 3 to 6% by mass. Bacterial proliferation is suppressed during hydrolysis of cellulose, and the ethanol concentration achieved in ethanol fermentation of a saccharified solution is increased, so that the distillation load is reduced. The ethanol added at the time of hydrolysis can be collected during distillation of the alcoholic fermentation liquid and reused.

## Description

### Technical Field

The present invention relates to a method used to produce ethanol (bioethanol) from sugars by alcoholic fermentation and particularly to a method for producing bioethanol by hydrolyzing cellulosic biomass with a hydrolytic enzyme.

### Background Art

In the context of biomass energy applications, there is an attempt to produce a saccharified solution by hydrolyzing cellulose or hemicellulose, which is a major constituent of plants, and obtain ethanol by subjecting the sugars of the saccharified solution to alcoholic fermentation. The planned goal of this attempt is the use of the obtained ethanol as fuel, such as, typically, the use as part of an automotive fuel mixture or as an alternative to gasoline.

Major constituents of plants include cellulose (a polymer of glucose which is a C6 sugar containing six carbon atoms), hemicellulose (a polymer of a C5 sugar containing five carbon atoms and a C6 sugar), lignin, and starch. Ethanol is produced by using as a raw material a sugar such as a C5 sugar, a C6 sugar, or an oligosaccharide which is a combination of C5 and C6 sugars and exposing the raw material to the fermentation activity of a microorganism such as a yeast fungus.

The following three methods are being considered for industrial use in hydrolysis of cellulosic biomass such as cellulose or hemicellulose into sugars: 1) a method in which cellulosic biomass is hydrolyzed by oxidizing power of a strong acid such as sulfuric acid; 2) a method in which cellulosic biomass is hydrolyzed with an enzyme; 3) a method utilizing the oxidizing power of supercritical water or subcritical water. Among these methods, the enzymatic hydrolysis method 2), although requiring a longer hydrolysis time than the other hydrolysis methods, has the advantages of involving a lower production equipment cost and a lower running cost, allowing the process to proceed at normal temperature and pressure, and having a lower likelihood of excessive sugar hydrolysis.

Biomass has a low specific gravity and is bulky. Hence, an increased slurry concentration causes a considerably poor flowability, which leads to difficult handling such as difficult transfer through pipes. An increased slurry concentration further gives rise to insufficient contact with solvents and an enzyme, resulting in a reduction in saccharification yield. Thus, a high sugar concentration of a saccharified solution becomes difficult to achieve, and the concentration of the saccharified solution to be subjected to a fermentation step is inevitably lowered. This leads to a low ethanol concentration of a fermentation liquid and an increase in distillation energy required in a distillation step. On the other hand, if the slurry concentration is reduced to allow easy handling, the sugar concentration of the saccharified solution is lowered, so that the load on the distillation step subsequent to the fermentation step is further increased.

Patent Literature 1 discloses an ethanol production method adapted to reduce the load on the distillation step, the method including saccharifying lignocellulose with an enzyme, then concentrating the resulting saccharified solution, and subjecting the concentrated saccharified solution to alcoholic fermentation.

### Citation List

### Patent Literature

PTL 1: Japanese Laid-Open Patent Application Publication No. 2011-45277

### Summary of Invention

### Technical Problem

When a saccharified solution is concentrated as disclosed in Patent Literature 1, a concentration device such as a membrane concentration device is needed, and the equipment cost is increased. Further, concentrating a saccharified solution requires a lot of time, thus causing an increase in the time for which the solution is retained in storage equipment and hence an increase in the risk of bacterial proliferation. If bacteria proliferate, sugars in the saccharified solution are consumed by the bacteria, or the efficiency of ethanol conversion by a yeast in the fermentation step is reduced. This leads to an increase in distillation load and a decrease in ethanol production.

When cellulose contained in biomass is hydrolyzed with a hydrolytic enzyme such as cellulase, it is ideal to prepare an aqueous solution containing cellulose at as high a concentration as possible and subject the aqueous solution to hydrolysis to produce a highly concentrated saccharified solution. However, when the solids concentration (biomass concentration) in the reaction vessel is high at the beginning of hydrolysis of cellulose, stirring of the contents of the reaction vessel is difficult due to the high viscosity of the contents. The use of a stirring device capable of applying high stirring power entails increases in equipment cost and operation cost.

An object of the present invention is to provide a bioethanol production method using lignocellulosic biomass as a raw material, the method being adapted to increase the ethanol concentration of a fermentation liquid obtained in the fermentation step and reduce the distillation load without having to use specialized equipment.

### Solution to Problem

As a result of intensive studies, the present inventors have found that the following advantages can be obtained by adding ethanol at a certain concentration when, in a cellulose hydrolysis step, a solid residue from which hemicellulose has been removed and an aqueous solution containing a cellulose-hydrolyzing enzyme are mixed and stirred in a reaction vessel: bacterial proliferation is suppressed to increase the amount of sugar production even if the enzymatic hydrolysis of cellulose is performed for a long time; and the ethanol concentration of a fermentation liquid resulting from alcoholic fermentation is increased. Based on these findings, the inventors have completed the present invention.

In particular, the present invention relates to a bioethanol production method using cellulosic biomass as a raw material, the method including the steps of:
A) hydrolyzing hemicellulose contained in the cellulosic biomass by hot water treatment or with a hemicellulose-hydrolyzing enzyme;
B) mixing a solid residue, from which hemicellulose has been removed, with an aqueous solution containing a cellulose-hydrolyzing enzyme in a reaction vessel, and then stirring the resulting mixture with a stirring device disposed in an interior of the reaction vessel to solubilize the solid residue from which hemicellulose has been removed;
C) continuing the stirring with the stirring device to allow a cellulose hydrolysis reaction to proceed in the reaction vessel until the hydrolysis reaction is completed;
D) withdrawing a slurry obtained as a result of the hydrolysis reaction from the reaction vessel;
E) subjecting the slurry obtained in the step D to alcoholic fermentation; and
F) distilling a fermentation liquid obtained as a result of the alcoholic fermentation to collect ethanol, wherein
in the step B, ethanol is added in an amount of 3 to 6% by mass based on the amount of the aqueous solution in the reaction vessel.

Preferably, part of ethanol collected in the step F is used as ethanol to be added in the step B.

Preferably, the reaction vessel has a flat, conical, or dished bottom surface, and the stirring device includes a plurality of impellers disposed at least in the interior of the reaction vessel.

Preferably, the stirring device includes an impeller selected from the group consisting of a helical ribbon impeller, a double helical ribbon impeller, a Maxblend impeller, an inclined paddle impeller, and an anchor impeller.

Preferably, in the bioethanol production method of the present invention, the step B and the step C are carried out in different reaction vessels, respectively, and after the step B, the contents of the reaction vessel in which the step B was carried out are withdrawn and delivered to the reaction vessel in which the step C is to be carried out.

The bioethanol production method of the present invention may further include a step A1 of removing lignin contained in the cellulosic biomass before the step B.

### Advantageous Effects of Invention

With the present invention, the distillation load can be reduced to lower the production cost of ethanol. Further, bacterial proliferation in a saccharification step and a fermentation step can be prevented.

### Brief Description of Drawings

FIG. 1 shows a basic flow of a process of producing a saccharified solution from cellulosic biomass by an enzymatic technique.
FIGS. 2A and 2B are schematic configuration diagrams showing examples of a reaction vessel suitable for carrying out the step B.
FIG. 3 illustrates an ethanol production method of Embodiment 1.
FIG. 4 illustrates an ethanol production method of Embodiment 2.

### Description of Embodiments

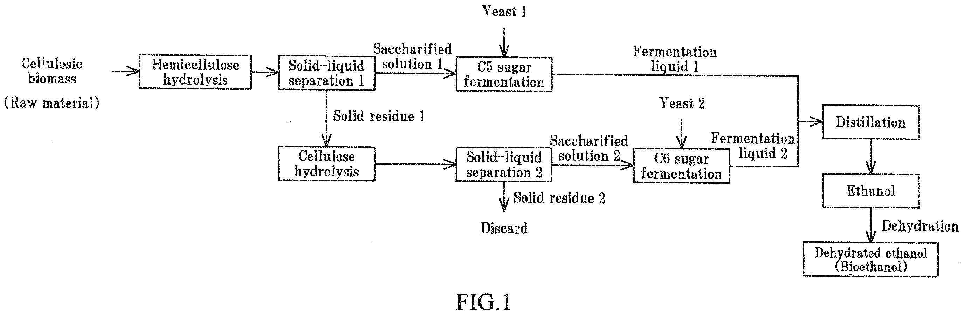

Embodiments of the present invention will be described with reference to the drawings. FIG. 1 shows a basic flow of a process of producing a saccharified solution from cellulosic biomass by an enzymatic technique.

### <Pre-treatment step>

First, cellulosic biomass (hereinafter referred to as "biomass") such as sugarcane bagasse is coarsely pulverized with a crusher or grinder. The cellulosic biomass is preferably pulverized into pieces with an average diameter of 10 to 100 mm. The coarsely pulverized biomass is subjected to cooking treatment using an aqueous solution of a metal hydroxide such as sodium hydroxide. This cooking treatment removes part of lignin contained in the bagasse and increases the reactivity of cellulose and hemicellulose with enzymes. The biomass subjected to the cooking treatment is neutralized with an acid.

Instead of the cooking treatment using an aqueous solution of a metal hydroxide, steam blasting treatment using a blasting treatment device may be performed at 200 to 240°C and 1.5 to 4 MPa for 1 to 15 min (preferably at 225 to 230°C and 2.5 to 3 MPa for 1 to 5 min) to accomplish saccharification of a hemicellulose component in the biomass and partial removal of lignin from the biomass. The biomass treated by the blasting treatment device (hereinafter referred to as "blasting-treated product") contains a C5 saccharified solution derived from hemicellulose, dissolved lignin, and a solid residue. When the C5 saccharified solution is to be subjected to hemicellulosic sugar fermentation separately from a C6 saccharified solution, the blasting-treated product is subjected to solid-liquid separation with means such as a filter press and separated into an blasting-treated solution and the solid residue. The solid residue may be washed with water as needed to collect sugars contained in the solid residue. When C5 sugar fermentation and C6 sugar fermentation are simultaneously performed, the solid-liquid separation may or may not be conducted.

The solid residue obtained through the blasting treatment is washed with water to remove sugars and dissolved lignin and then immersed in an aqueous ethanol solution having an ethanol concentration of 30% or more, preferably 50% or more, at normal temperature for a time of 0.5 to 48 hours, preferably 1 to 24 hours, to dissolve and remove lignin covering cellulose. After the ethanol immersion, the solid residue is subjected to ethanol removal treatment with mechanical means or by heating or heating under reduced pressure. When the aqueous ethanol solution used has a high concentration, the solid residue may be washed with water before the ethanol removal treatment.

### <Hemicellulose hydrolysis (saccharification)>

Hemicellulose contained in the cellulosic biomass is hydrolyzed using high-pressure hot water (hot water treatment) or hydrolyzed with a hemicellulose-hydrolyzing enzyme such as hemicellulase. Hydrolysis using high-pressure hot water at 140 to 180°C can hydrolyze hemicellulose into sugars (mainly C5 monosaccharides). When the biomass has a high hemicellulose content, treatment at high temperature causes excessive hydrolysis of the C5 monosaccharides into organic acids etc. Thus, the hydrolysis treatment is preferably carried out under relatively mild conditions.

It is considered preferable, when hemicellulose is hydrolyzed using high-pressure hot water, to add an acid such as phosphoric acid or hydrochloric acid as a catalyst. When hemicellulose is hydrolyzed with a hemicellulose-hydrolyzing enzyme, a commercially-available enzyme may be used, or a microorganism that produces hemicellulase may be used.

### <Solid-liquid separation 1>

The hydrolysis of hemicellulose is followed by solid-liquid separation 1 to separate a solid residue 1 and a saccharified solution 1 (C5 saccharified solution) from each other.

### <C5 sugar fermentation>

A yeast is added to the saccharified solution 1, in which hemicellulosic sugar fermentation proceeds to give an alcohol (fermentation liquid 1).

### <Cellulose hydrolysis (saccharification)>

To the solid residue 1 are added water and a cellulose-hydrolyzing enzyme such as cellulase, and thus cellulose contained in the solid residue 1 is hydrolyzed. When cellulose is hydrolyzed with a cellulose-hydrolyzing enzyme, a commercially-available enzyme may be used, or a microorganism that produces cellulase may be used.

### <Solid-liquid separation 2>

The hydrolysis of cellulose is followed by solid-liquid separation 2 to separate a solid residue 2 and a saccharified solution 2 (C6 saccharified solution) from each other. The solid residue is washed with water as needed to collect C6 sugars and then discarded.

### <C6 sugar fermentation>

A yeast is added to the saccharified solution 2, in which cellulosic sugar fermentation proceeds to give an alcohol (fermentation liquid 2).

While in FIG. 1 the C5 sugar fermentation and C6 sugar fermentation are carried out independently of each other, the saccharified solution 1 and saccharified solution 2 may be mixed, and the C5 sugar fermentation and C6 sugar fermentation may be allowed to proceed simultaneously in the mixture of the saccharified solutions.

### <Distillation and dehydration>

The alcoholic fermentation liquids (fermentation liquid 1 and fermentation liquid 2) resulting from alcoholic fermentation of the C5 and C6 saccharified solutions are delivered to a distillation device, in which the fermentation liquids are distilled to give ethanol. The resulting ethanol is dehydrated with a known ethanol dehydration agent or ethanol dehydration device, and the dehydrated ethanol is shipped as an end product (bioethanol).

In FIG. 1, the solid-liquid separation 1 or solid-liquid separation 2 may be skipped, and the hemicellulosic sugar fermentation and cellulosic sugar fermentation may be performed with the solid residue remaining in the saccharified solution.

### (Step A)

Hereinafter, the steps of the saccharified solution production method of the present invention will be described. First, cellulosic biomass is coarsely pulverized, and then hemicellulose contained in the pulverized cellulosic biomass is hydrolyzed by hot water treatment or with a hemicellulose-hydrolyzing enzyme. The step A is followed by solid-liquid separation using a solid-liquid separation device to separate a C5 saccharified solution and a solid residue from each other.

### (Step B)

Next, the solid residue, from which hemicellulose has been removed, is mixed with an aqueous solution containing a cellulose-hydrolyzing enzyme in a reaction vessel. The bottom surface of the reaction vessel is flat, conical, or dished. The material, dimensions, and other properties of the vessel can be selected as appropriate depending on the amount of the cellulosic biomass to be processed.

In the interior of the reaction vessel, a plurality of impellers of a stirring device are disposed at least in a region where the impellers can contact the contents of the vessel. The ratio of the rotation diameter of the impellers to the inner diameter of the reaction vessel is preferably large. Ideally, the rotation diameter is 70% or more of the inner diameter of the reaction vessel. Each impeller of the stirring device is preferably a helical ribbon impeller, a double helical ribbon impeller, a Maxblend impeller, an inclined paddle impeller, or an anchor impeller. The plurality of impellers of the stirring device may be of the same or different types.

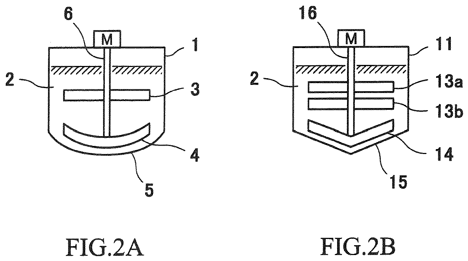

FIGS. 2A and 2B are schematic configuration diagrams showing examples of the reaction vessel suitable for carrying out the step B. A reaction vessel 1 shown in FIG. 2A includes impellers 3 and 4 and has a dished bottom surface 5. A rotary shaft 6 is rotated by a motor M mounted on the top surface of the reaction vessel 1. A reaction vessel 11 shown in FIG. 2B includes impellers 13a, 13b, and 14. The impellers 13a and 13b are of the same type. The reaction vessel 11 has a conical bottom surface 15. A rotary shaft 16 is rotated by a motor M mounted on the top surface of the reaction vessel 11.

Preferably, ethanol and the aqueous solution containing the cellulose-hydrolyzing enzyme are first introduced into the reaction vessel, and then the solid residue from which hemicellulose has been removed is introduced stepwise. Necessary amounts of water and ethanol may be first introduced into the vessel, and then the cellulose-hydrolyzing enzyme may be added and mixed with the water to prepare the aqueous solution of the cellulose-hydrolyzing enzyme. The amount of ethanol added is 3 to 6% by mass based on the amount of the aqueous solution in the reaction vessel. That is, ethanol is added in an amount such that the ethanol concentration of the liquid in the reaction vessel is 3 to 6% by mass.

Preferably, the solid residue is introduced bit by bit while the aqueous solution is stirred by the impellers of the stirring device. The solids concentration in the reaction vessel is adjusted so that the final concentration is 15 to 30% by mass. Afterwards, the contents of the vessel are stirred by the impellers of the stirring device for 5 minutes to 12 hours. Through this process, the solid residue (cellulosic biomass) from which hemicellulose has been removed is solubilized. In the step B, the contents of the reaction vessel are preferably adjusted to a temperature of 20 to 90°C.

In the step B, once the solids concentration of the contents of the reaction vessel reaches a predetermined concentration (15 to 30% by mass) and the cellulosic biomass is solubilized, the aqueous solution containing the cellulose-hydrolyzing enzyme and the solid residue from which hemicellulose has been removed may be continuously introduced into the reaction vessel so that the solids concentration is kept within the predetermined concentration range. Further, the liquid in the reaction vessel may be removed in such a manner that the amount of the removed liquid corresponds to the amount of the aqueous solution and solid residue which are continuously introduced, and the removed liquid may be delivered to another reaction vessel in which the step C is carried out. This allows the step B to be carried out continuously.

The step B is preferably preceded by a step A1 of subjecting the solid residue, from which hemicellulose has been removed, to the cooking treatment or steam explosion treatment described above and thereby removing lignin from the solid residue. The step A1 may be performed before the step A or between the step A and step B.

### (Step C)

Next, the contents of the reaction vessel (a mixture of the solid residue from which hemicellulose has been removed and the aqueous solution containing the cellulose-hydrolyzing enzyme) are stirred by the stirring device to allow a hydrolysis reaction to proceed until most of contained cellulose is converted to glucose (in other words, until the cellulose hydrolysis reaction of the contents of the reaction vessel is completed). The step C is continued for a time of 5 minutes to 120 hours while the contents of the reaction vessel are adjusted to a temperature of 20 to 90°C. Through the step C, cellulose contained in the solid residue is hydrolyzed, and the contents of the reaction vessel are formed into a slurry.

In the present invention, ethanol is added to the reaction vessel contents in the step B. This addition of ethanol can prevent bacteria from proliferating in the reaction vessel contents during the steps B and C. Consequently, consumption of sugars by the bacteria can be prevented, and the efficiency of the subsequent alcoholic fermentation of the saccharified solution can be improved.

### (Step D)

After the step C, the slurry is withdrawn from the reaction vessel. The withdrawn slurry is subjected to solid-liquid separation as needed to separate the slurry into a solid residue and a C6 saccharified solution. The C6 saccharified solution alone or mixed with the C5 saccharified solution is subjected to alcoholic fermentation as a raw material for bioethanol.

The steps B and C may be carried out in a single reaction vessel. Alternatively, the steps B and C may be carried out in different reaction vessels, respectively. In this case, after the step B, the contents of the reaction vessel in which the step B was carried out may be withdrawn from the vessel and delivered by means such as a pump to a reaction vessel in which the step C is to be carried out. The step C requires a longer time than the step B. Thus, when a large quantity of cellulosic biomass is processed continuously, it is preferable to carry out the steps B and C in different reaction vessels. In this case, the reaction vessel for the step C may have the same structure as the reaction vessel for the step B, but is preferably larger in size than the reaction vessel for the step B.

Once a steady state is reached in the step C and the vessel contents are formed into a slurry, the slurry may be withdrawn from the reaction vessel for the step C in such a manner that the amount of the withdrawn slurry corresponds to the volume of the solubilized cellulosic biomass and the cellulose-hydrolyzing enzyme-containing aqueous solution which are delivered from the reaction vessel for the step B. In this case, a saccharified solution can be produced continuously while the solids concentration of the slurry in the step C is kept within in a certain range. The reaction vessel for the step B may be small.

### (Step E)

In the step D, the slurry (saccharified slurry) withdrawn from the reaction vessel in which the step C was carried out is delivered to a fermentation chamber. After the step A, the C5 saccharified solution separated from the solid residue may alone be subjected to ethanol fermentation. Alternatively, the C5 saccharified solution may be delivered to the fermentation chamber together with the slurry withdrawn from the reaction vessel in which the step C was carried out, and the C5 saccharified solution and the slurry may be subjected together to alcoholic fermentation. In the fermentation chamber, C6 sugars (and/or C5 sugars) undergo ethanol fermentation under the activity of a yeast, thus giving ethanol. In the step E, which is a fermentation step, a known fermentation technique can be employed. Through the step E, C6 sugars (and/or C5 sugars) contained in the saccharified slurry are converted to ethanol. The saccharified slurry may be subjected to solid-liquid separation using a solid-liquid separation device such as a centrifuge, and only the saccharified solution may be delivered to the fermentation chamber and subjected to the step E. The residue discharged from the solid-liquid separation device is discarded as appropriate.

The step E is preferably accomplished by holding the fermentation liquid in the fermentation chamber at 30 to 40°C for about 24 hours. While in a fermentation step of a common process the ethanol concentration of the fermentation liquid at the end of the fermentation step is about 1 to 4% by mass, the ethanol concentration of the fermentation liquid at the end of the fermentation step in the present invention is about 5 to 10% by mass because of the addition of ethanol in the step B. Considering adverse effects on the yeast, the ethanol concentration at the end of the step E is preferably adjusted to 15% by mass or less.

### (Step F)

After the step E, the fermentation liquid is delivered to a distillation device. As a result of distillation, distilled ethanol (bioethanol) is obtained. The bioethanol obtained in the step F is emptied of components other than solids and ethanol. In the step F, which is a distillation step, a distillation technique known for use in production of distilled liquors can be employed. In the present invention, since the ethanol concentration of the fermentation liquid at the end of the step E is higher than usual, the load on the distillation device can be reduced.

### (Collection of ethanol)

The bioethanol obtained in the step F is subjected, as necessary, to a high-class treatment such as dehydration and then transferred to and stored in storage equipment such as a storage tank. Part of the bioethanol can be used as ethanol to be added in the step B. Thus, in the present invention, ethanol added in the step B can be collected afterwards in the step F and reused as ethanol to be added in the step B.

### [Embodiment 1]

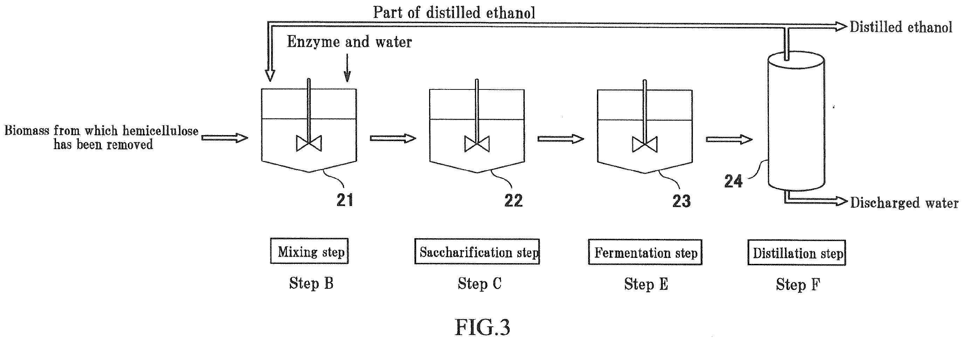

FIG. 3 illustrates an ethanol production method of Embodiment 1. In the ethanol production method of Embodiment 1, the steps B, C, and E are carried out in a mixing chamber 21, a saccharification chamber 22, and a fermentation chamber 23, respectively. In a steady state, part of distilled ethanol obtained in a distillation device 24 is delivered to the chamber 21 in which the step B is carried out. The distilled ethanol added to the chamber 21 is collected afterwards from the distillation device 24.

### [Embodiment 2]

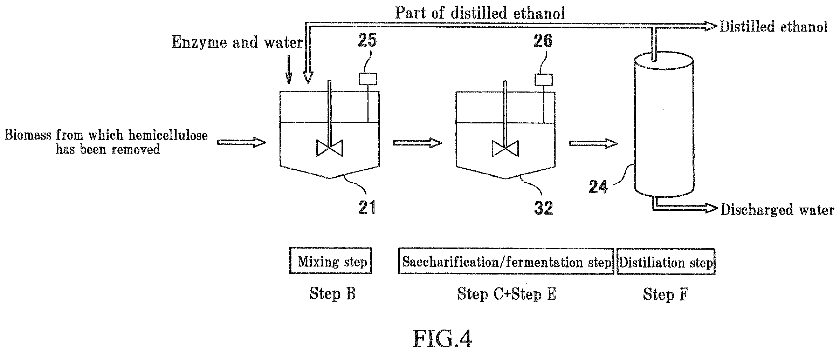

FIG. 4 illustrates an ethanol production method of Embodiment 2. The ethanol production method of Embodiment 2 is the same as that of Embodiment 1, except that the step C (saccharification step) and the step E (fermentation step) are performed successively in a single chamber (saccharification/fennentation chamber 32).

In Embodiment 2, ethanol concentration meters 25 and 26 are provided for the mixing chamber 21 and saccharification/fermentation chamber 32, respectively, and each of the meters measures the ethanol concentration of the contents of the chamber. The amount of the distilled ethanol to be added to the mixing chamber 21 can be adjusted based on observation of the values measured by the ethanol concentration meters 25 and 26.

### Industrial Applicability

The method of the present invention is beneficial as a bioethanol production method using cellulosic biomass as a raw material.

### Reference Signs List

- 1, 11:: reaction vessel
- 2:: contents of reaction vessel
- 3:: impeller (upper impeller)
- 4:: impeller (lower impeller)
- 5:: dished bottom surface
- 6:: rotary shaft of stirring device
- 13a:: impeller (upper impeller)
- 13b:: impeller (central impeller)
- 14:: impeller (lower impeller)
- 15:: conical bottom surface
- 21:: mixing chamber
- 22:: saccharification chamber
- 23:: fermentation chamber
- 24:: distillation device
- 25, 26:: ethanol concentration meter
- 32:: saccharification/fermentation chamber

## Claims

1. A bioethanol production method using cellulosic biomass as a raw material, comprising the steps of:
A) hydrolyzing hemicellulose contained in the cellulosic biomass by hot water treatment or with a hemicellulose-hydrolyzing enzyme;
B) mixing a solid residue, from which hemicellulose has been removed, with an aqueous solution containing a cellulose-hydrolyzing enzyme in a reaction vessel, and then stirring the resulting mixture with a stirring device disposed in an interior of the reaction vessel to solubilize the solid residue from which hemicellulose has been removed;
C) continuing the stirring with the stirring device to allow a cellulose hydrolysis reaction to proceed in the reaction vessel until the hydrolysis reaction is completed;
D) withdrawing a slurry obtained as a result of the hydrolysis reaction from the reaction vessel;
E) subjecting the slurry obtained in the step D to alcoholic fermentation; and
F) distilling a fermentation liquid obtained as a result of the alcoholic fermentation to collect ethanol, wherein
in the step B, ethanol is added in an amount of 3 to 6% by mass based on the amount of the aqueous solution in the reaction vessel.

2. The bioethanol production method according to claim 1, wherein part of ethanol collected in the step F is used as ethanol to be added in the step B.

3. The bioethanol production method according to claim 1 or 2, wherein
the reaction vessel has a flat, conical, or dished bottom surface, and
the stirring device includes a plurality of impellers disposed at least in the interior of the reaction vessel.

4. The bioethanol production method according to any one of claims 1 to 3, wherein the stirring device includes an impeller selected from the group consisting of a helical ribbon impeller, a double helical ribbon impeller, a Maxblend impeller, an inclined paddle impeller, and an anchor impeller.

5. The bioethanol production method according to any one of claims 1 to 4, wherein
the step B and the step C are carried out in different reaction vessels, respectively, and
after the step B, the contents of the reaction vessel in which the step B was carried out are withdrawn and delivered to the reaction vessel in which the step C is to be carried out.

6. The bioethanol production method according to any one of claims 1 to 5, further comprising a step A1 of removing lignin contained in the cellulosic biomass before the step B.
